Europäisches Patentamt

European Patent Office

Office européen des brevets

(19)

(11) **EP 0 493 686 B1**

(12) **EUROPEAN PATENT SPECIFICATION**

(45) Date of publication and mention
of the grant of the patent:
**12.02.1997 Bulletin 1997/07**

(51) Int Cl.$^6$: **C12P 21/08**, C12N 5/20,
C12N 15/06, A61K 39/395

(21) Application number: **91120585.4**

(22) Date of filing: **29.11.1991**

(54) **Monoclonal antibodies to glycolipid carbohydrate chains**

Monoklonale Antikörper gegen Glykolipid-Karbohydratketten

Anticorps monoclonaux contre les chaînes hydrocarbonées glycolipidiques

(84) Designated Contracting States:
**AT BE CH DE DK ES FR GB GR IT LI LU NL SE**

(30) Priority: **30.11.1990 JP 334659/90**

(43) Date of publication of application:
**08.07.1992 Bulletin 1992/28**

(73) Proprietor: **Kyowa Hakko Kogyo Co., Ltd.
Chiyoda-ku, Tokyo 100 (JP)**

(72) Inventors:
• **Hanai, Nobuo
Sagamihara-shi, Kanagawa (JP)**
• **Ohta, So
Machida-shi, Tokyo (JP)**

(74) Representative: **Kinzebach, Werner, Dr. et al
Patentanwälte
Reitstötter, Kinzebach und Partner
Postfach 86 06 49
81633 München (DE)**

(56) References cited:
**EP-A- 0 234 122          EP-A- 0 280 209**

• **BIOCHIMICA ET BIOPHYSICA ACTA vol. 837, no. 3, 1985, AMSTERDAM, THE NETHERLANDS pages 349 - 353; T. BRODIN ET AL.: 'Mouse monoclonal antibodies with specificity for the melanoma-associated ganglioside disialyllactosylceramide (GD3) also react with the structural analogue disialylparagloboside.'**

• **ACTA NEUROPATHOLOGICA vol. 79, no. 3, 1989, BERLIN, GERMANY pages 317 - 325; X.HE ET AL.: 'GD3 expression by cultured human tumor cells of neuroectodermal origin.'**

• **PROCEEDINGS OF THE NATIONAL ACADEMY OF SCIENCES OF USA vol. 82, no. 24, December 1985, WASHINGTON DC, USA pages 8653 - 8657; Z. STEPLEWSKI ET AL.: 'Isolation and characterization of anti-monosialoganglioside monoclonal antibody 19-9 class-switch variants.'**

• **CLINICAL IMMUNOLOGY AND IMMUNOPATHOLOGY vol. 45, no. 2, November 1987, NEW YORK, USA pages 214 - 229; S. WELT ET AL.: 'Immune and nonimmune effector functions of IgG3 mouse monoclonal antibody R24 detecting the disialoganglioside GD3 on the surface of melanoma cells.'**

• **PROCEEDINGS OF THE NATIONAL ACADEMY OF SCIENCES OF USA vol. 82, no. 15, August 1985, WASHINGTON DC, USA pages 5155 - 5159; D. CHERESH ET AL.: 'Disialoganglioside GD3 on human melanoma serves as a relevant target antigen for monoclonal antibody-mediated tumor cytolysis.'**

Remarks:
The file contains technical information submitted after the application was filed and not included in this specification

EP 0 493 686 B1

**Description**

FIELD OF THE INVENTION

This invention relates to monoclonal antibodies belonging to the subclass IgG3 and reactive with at least one GD3 ganglioside and with 3',8'-LD1.

BACKGROUND OF THE INVENTION

Gangliosides, which constitute a class of cell membrane constituent glycolipids, are molecules composed of a carbohydrate, or oligosaccharide, chain, which is a hydrophilic side chain, and a sphingosine and a fatty acid, which are hydrophobic moieties.

It is known that ganglioside expression differs among cells, organs, and animal species. Furthermore, it has gradually become clear that gangliosides expressed in the process of oncogenic transformation of cells show changes in quantity and quality [Cancer Res., 45, 2405 (1985)].

For instance, it is reported that such gangliosides as GD2, GD3 and GM2, which are hardly detected in normal cells, are expressed in an abnormal manner as cancer antigens in neuroblastoma, lung small cell carcinoma, glioma and melanoma, which are neuroectodermal tumors and said to be highly malignant [J. Exp. Med., 155, 1133 (1982); J. Biol. Chem., 257, 12752 (1982); Cancer Res., 47, 225 (1987); ibid., 47, 1098 (1987); ibid., 45, 2642 (1985); Proc. Natl. Acad. Sci. U.S.A., 80, 5392 (1983)].

For the analysis and treatment of such tumors, monoclonal antibodies reactive with the ganglioside GD2 [Cancer Res., 47, 1098 (1987); ibid., 45, 2642 (1985); Proc. Natl. Acad. Sci. U.S.A., 79, 7629 (1982)) and monoclonal antibodies reactive with the ganglioside GM2 [Cancer Res., 46, 4116 (1986); ibid., 48, 6154 (1988); Proc. Natl. Acad. Sci. U.S.A., 80, 5392 (1983)] have been produced.

GD3 ganglioside species contain the carbohydrate chain NeuAc$\alpha$2→8NeuAc$\alpha$2→3Gal, NeuAc$\alpha$2→8NeuGc$\alpha$2→3Gal, NeuGc$\alpha$2→8NeuAc$\alpha$2→3Gal or NeuGc$\alpha$2→8NeuGc$\alpha$2→3Gal at the nonreducing end. These are expressed frequently in neuroectodermal tumors, in particular in melanoma. So far, five monoclonal anti-ganglioside GD3 antibodies belonging to the class IgM or subclass IgG3 have been produced and reported to be reactive to the ganglioside GD3 [Int. J. Cancer, 29, 269 (1982); J. Biol. Chem., 257, 12752 (1982); Cancer Res., 47, 225 (1987); Acta Neuropathol., 79, 317 (1989); Proc. Natl. Acad. Sci. U.S.A., 77, 6114 (1980); J. Exp. Med., 155, 1133 (1982); Proc. Natl. Acad. Sci., U.S.A., 81, 5767 (1984)].

Treatment of patients with melanoma has been attempted using a monoclonal anti-ganglioside GD3 antibody belonging to the subclass IgG3 [Eur. J. Cancer Clin. Oncol., 24, Suppl. 2, S65 (1988); Proc. Natl. Acad. Sci. U.S.A., 82, 1242 (1985)]. However, the therapeutic effects are not satisfactory as yet.

On the other hand, it is reported that the ganglioside 3',8'-LD1, which has the same carbohydrate chain as that of GD3 at the nonreducing end, is expressed in glioma, one of neuroectodermal tumors. The report also deals with a monoclonal antibody which belongs to the IgM class and reacts with 3',8'-LD1 [Acta Neuropathol., 79, 317 (1989)]. Accordingly, 3',8'-LD1 can be regarded as being important as one of cancer antigens.

Furthermore, a monoclonal antibody belonging to the IgM class and showing cross reactivity to both of the antigenic gangliosides GD3 and 3',8'-LD1 is known [Acta Neuropathol., 79, 317 (1989)]. There are no known antibodies of the IgG class which react with GD3 and 3',8'-LD1.

Brodin et al. disclose in Biochimica et Biophysica Acta 837 (1985) 349-553, mouse monoclonal antibodies 2B2 and 1F4, subclass IgG3, with specificity for ganglioside GD3.

Monoclonal antibodies reacting with the gangliosides GD3 and 3',8'-LD1 and belonging to the IgG class, if obtained, would be useful in the treatment of carcinomas of neuroectodermal origin.

SUMMARY OF THE INVENTION

It has now been found that certain monoclonal antibodies of the IgG class as produced by hybridomas established by the present inventors using a GD3 ganglioside or GD3-expressing cells as an immunogen can react simultaneously with the gangliosides GD3 and 3',8'-LD1 and have cytotoxic activity, hence are useful in cancer treatment.

BRIEF DESCRIPTION OF THE DRAWINGS

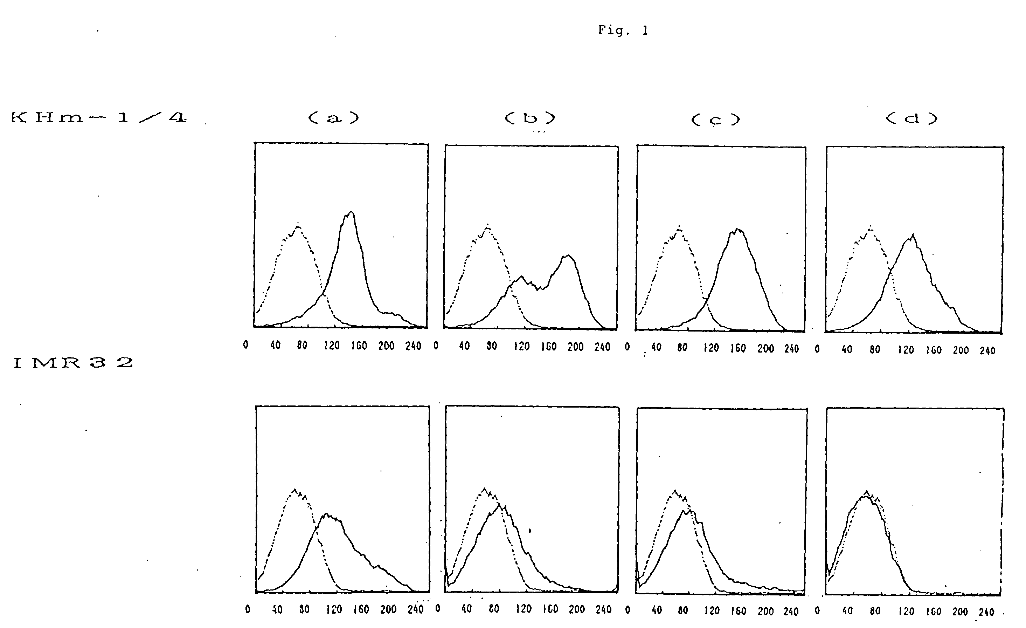

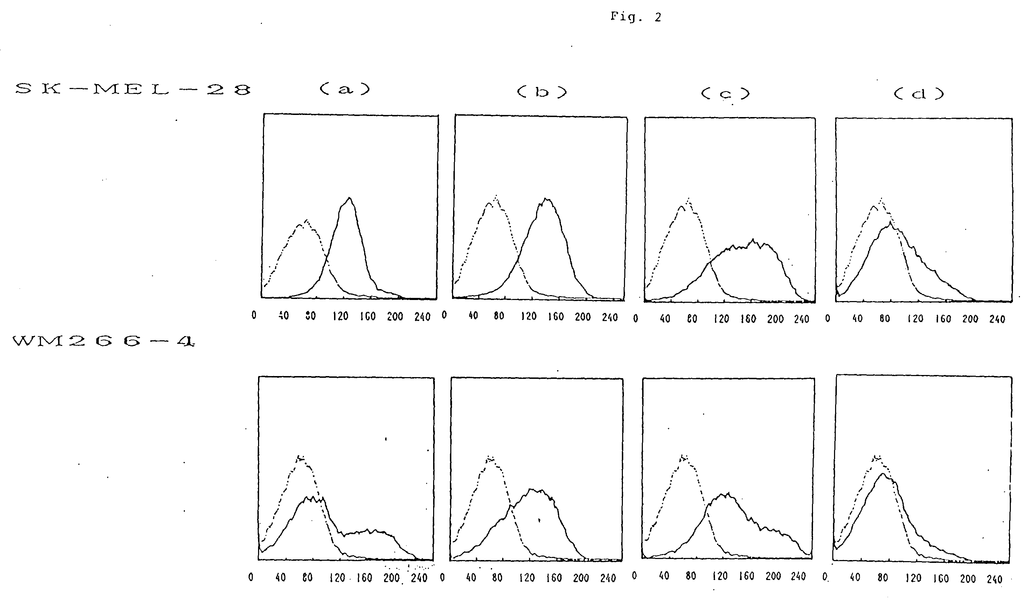

Fig. 1 and Fig. 2 show the results of Example 2 as obtained by the immunofluorescence method, with the cell count on the ordinate and the fluorescence intensity on the abscissa. The data in row (a) show the reactivity of KM-641 with ganglioside GD3-positive KHm-1/4, IMR32, SK-MEL-28 and WM-266-4 cells; those in row (b) show the reactivity of KM-643, those in row (c) show that of KM-644, and those in row (d) show that of R24.

Fig. 3 shows the change in tumor volume over time in days of the transplanted tumors determined in Example 5. In Fig. 3, open circle stands for the control group to which AMC-462 (200 µg/animal) was administered, closed circle stands for the test group to which KM-641 (200 µg/animal) was administered from the day of the transplantation and open square stands for the test group to which KM-641 (200 µg/animal) was administered from the seventh day after the transplantation.

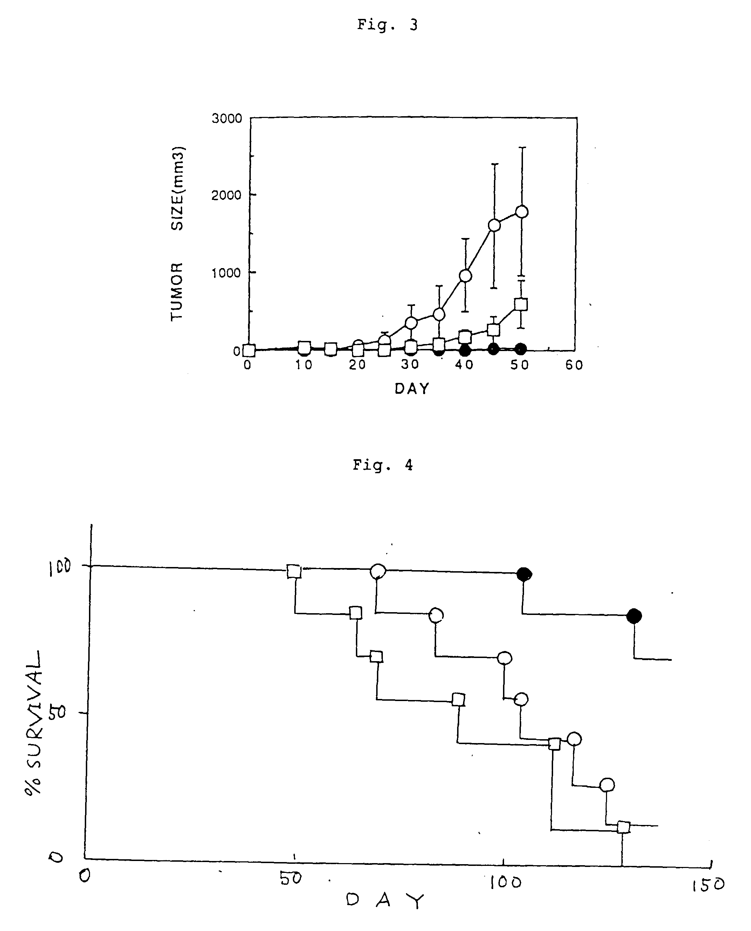

Fig. 4 shows the survival rate of mice determined in Example 5. The symbols in Fig. 4 have the same meaning as defined in Fig. 3.

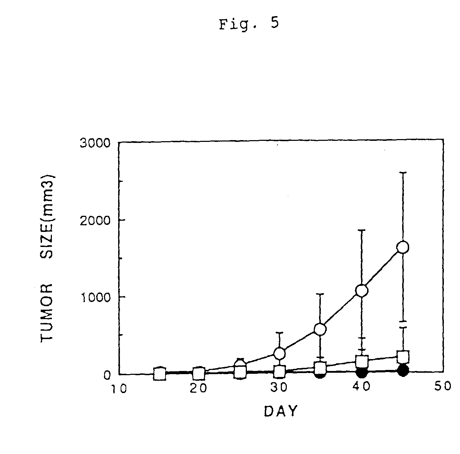

Fig. 5 shows the change in tumor volume over time in days of the transplanted tumors determined in Example 5. In Fig. 5, open circle stands for the control group to which AMC-462 (200 µg/animal) was administered, closed circle stands for the test group to which KM-641 (200 µg/animal) was administered, and open square stands for the test group to which KM-641 (100 µg/animal) was administered.

## DETAILED DESCRIPTION OF THE INVENTION

The GD3 ganglioside to be used in the present invention is selected from among GD3 ganglioside species having the NeuAc$\alpha$2$\rightarrow$8NeuAc$\alpha$2$\rightarrow$3Gal, NeuAc$\alpha$2$\rightarrow$8NeuGc$\alpha$2$\rightarrow$3Gal, NeuGc$\alpha$2$\rightarrow$8NeuAc$\alpha$2$\rightarrow$3Gal or NeuGc$\alpha$2$\rightarrow$8NeuGc$\alpha$2$\rightarrow$3Gal carbohydrate chain.

The monoclonal antibodies according to the invention can be produced by preparing hybridomas by fusion of splenocytes (spleen cells) of a mouse immunized with the GD3 ganglioside or cancer cells in which the GD3 ganglioside has been expressed, on one hand and, on the other, a mouse myeloma cell line, selecting a hybridoma which produces a monoclonal antibody reactive with the GD3 ganglioside and 3',8'-LD1, culturing said hybridoma in a medium or administering said hybridoma to mice to thereby grow the same in the ascites fluid of said mice, and recovering the thus-produced monoclonal antibody from the culture or ascites fluid.

As typical examples of the hybridoma capable of producing a monoclonal antibody of the present invention, there may be mentioned the hybridomas KM-641, KM-643 and KM-644, which have been deposited at the Fermentation Research Institute, Agency of Industrial Science and Technology, Japan of 1-3, Higashi 1-chome, Tsukuba-shi, Ibaraki, Japan under the deposit numbers FERM BP-3116, FERM BP-3117 and FERM BP-3118, respectively, since September 27, 1990 in accordance with the Budapest Treaty. The monoclonal antibodies produced by the respective hybridomas are designated as KM-641, KM-643 and KM-644.

A method of producing the monoclonal antibodies of this invention is described below in further detail.

### (1) Immunization of animals and preparation of antibody-producing cells

Mice of 3 to 20 weeks of age are immunized with a GD3 ganglioside antigen, and antibody-producing cells are prepared from the spleen, lymph node or peripheral blood of the animals.

The method of immunization comprises, for example, administering the GD3 ganglioside borne on a carrier, such as Escherichia coli or other bacterial cells, subcutaneously, intravenously or intraperitoneally to the animals, administering cancer cells in which the GD3 ganglioside has been expressed to the animals in the same manner, or administering the GD3 ganglioside borne, together with lipid A, on liposomes made of a lipid, such as a phospholipid or cholesterol, to the animals in the same manner. Such administration is performed 5 to 10 times at one- to two-week intervals following the first administration. Three to seven days after each administration, blood is sampled from the venous plexus of the fundus of the eye, and the serum derived from the sample blood is tested, for example, by the enzyme immunoassay technique mentioned below [cf. the monograph "Koso Men-eki Sokuteiho (ELISA)", published by Igaku Shoin, 1976] as to whether it is reactive with the GD3 ganglioside.

Enzyme immunoassay (ELISA):

An ethanol solution containing a GD3 ganglioside, a phospholipid and cholesterol is distributed in 20-µl portions (20 picomole of ganglioside per well) into wells of a 96-well plate for EIA (Flow Laboratories). After air-drying, PBS (phosphate buffered saline; prepared by dissolving 1.83 g of disodium phosphate, 0.21 g of monopotassium phosphate and 7.65 g of sodium chloride in 1 liter of distilled water; pH 7.2) containing 1% BSA (bovine serum albumin) (BSA-PBS) is distributed in 100- to 200-µl portions into the wells, and the plate is allowed to stand at 4°C for 10 to 30 hours for blocking the protein binding groups remaining on the plate. Then the BSA-PBS is discarded, the plate is washed well with deionized water or PBS, a dilution of the sample (mouse serum, hybridoma culture supernatant, or purified monoclonal antibody) in BSA-PBS is distributed, as a first antibody, in 100-µl portions into the wells, and the plate is allowed to stand at 4°C for 10 hours. The plate is washed once with deionized water and then six times with 2 M sodium chloride solution. Thereafter, a 400-fold dilution of rabbit anti-mouse immunoglobulin antibody-peroxidase conjugate (Dako) is distributed, as a second antibody, in 100-µl portions into the wells. The plate is then allowed to stand at room temperature for 2 hours.

After washing the plate well with PBS, an ABTS substrate solution [prepared by dissolving 550 mg of 2,2'-azinobis (3-ethylbenzothiazoline-6-sulfonic acid) diammonium salt in 1 liter of 0.1 M citrate buffer (pH 4.2) and adding, just prior to use, hydrogen peroxide in an amount of 1 μl/ml] is applied to the plate, and the color development on each well is measured in terms of the optical density at 415 nm ($OD_{415 \text{ nm}}$).

For submission for cell fusion, splenocytes are prepared by excising the spleen from the immunized mouse 3 to 4 days after the last immunogen administration. The spleen is cut to pieces in MEM (Nissui Pharmaceutical), and loosened with a pair of forceps. After centrifugation (1,200 rpm, 5 minutes), the supernatant is discarded, and the sediment is treated with Tris-ammonium chloride buffer (pH 7.65) for 1 to 2 minutes for eliminating erythrocytes. The remaining cells are washed three times with MEM and submitted for cell fusion.

(2) Preparation of myeloma cells

Mouse-derived established cell lines are used as the myeloma cells. Thus, for instance, the 8-azaguanine-resistant mouse (BALB/c-derived) myeloma cell lines P3-X63Ag8-U1 (P3-U1) [Current Topics in Microbiology and Immunology, 81, 1-7 (1978)] [European J. Immunology, 6, 511-519 (1976)], SP2/0-Ag14 (SP-2) [Nature, 276, 269-270 (1978)], P3-X63-Ag8653 (653) [J. Immunology, 123, 1548-1550 (1979)] and P3-X63-Ag8 (X63) [Nature, 256, 495-497 (1975)] may be used. These cell lines are subcultured in an 8-azaguanine medium [prepared by supplementing RPMI-1640 medium with glutamine (1.5 mM), 2-mercaptoethanol (5 x $10^{-5}$ M), gentamicin (10 μg/ml) and fetal calf serum (FCS) (CSL; 10%) and further supplementing the resulting normal medium with 8-azaguanine (15 μg/ml)]. Three to four days before cell fusion, subculture is performed in the normal medium to thereby ensure a cell number of not less than 2 x $10^7$ cells on the day of cell fusion.

(3) Cell fusion

The antibody-producing cells obtained as described under (1) and the myeloma cells obtained as described under (2) are respectively washed well with MEM medium or PBS, and mixed in a proportion of 5 to 10 antibody-producing cells per myeloma cell, and the mixture is subjected to centrifugation (1,200 rpm, 5 minutes). The supernatant is discarded and the sediment (cells) is loosened. To the cells are added 2 g of polyethylene glycol-1,000 (PEG-1,000) and a mixture of 2 ml of MEM and 0.7 ml of dimethyl sulfoxide in an amount of 0.2 to 1 ml/$10^3$ antibody-producing cells at 37°C with stirring. Several 1- to 2-ml portions of MEM are added at 1- to 2-minute intervals, and then the whole amount is made 50 ml by further addition of MEM. After centrifugation (900 rpm, 5 minutes), the supernatant is discarded and the cells are loosened gently. To the cells is added 100 ml of a normal medium (RPMI-1640 medium containing 10% FCS), and the cells are suspended in the medium by gentle drawing up into and discharging from a measuring pipette.

This suspension is distributed in 100-μl portions into wells of a 96-well incubation plate, and incubation is performed in a 5% $CO_2$ incubator at 37°C for 24 hours. HAT medium [prepared by supplementing normal medium with hypoxanthine ($10^{-4}$ M), thymidine (1.5 x $10^{-5}$ M) and aminopterin (4 x $10^{-7}$ M)] is added to the incubation plate in an amount of 100 μl/well and incubation is conducted for another 24 hours. Thereafter, 100 μl of the culture supernatant is discarded and the same volume of fresh HAT medium is added instead at 24-hour intervals for 2 days. Incubation at 37°C in the presence of 5% $CO_2$ is continued for 10 to 14 days.

For those wells in which fused cells grown and forming colonies are found, the supernatant (100 μl) is discarded and the same volume of HT medium (HAT medium minus aminopterin) is added instead. The medium replacement with fresh HT medium is repeated at 24-hour intervals for 2 days.

After 3 to 4 days of cultivation in HT medium, a portion of the culture supernatant is taken from each well and assayed for antibody titer against the ganglioside GD3 by the above-mentioned enzyme immunoassay or by immunohistological evaluation (ABC method) [cf. the monograph "Koso Kotai Ho (Enzyme-labeled Antibody Techniques)", page 100, published by Gakusai Kikaku, 1985]. Simultaneously, the reactivity with other gangliosides, such as GM3, is tested by the same method. The wells showing specific reactivity with the ganglioside GD3 are selected, and for the wells showing strong reactivity with the ganglioside GD3 but no reactivity with other gangliosides, such as GM3, cloning is repeated twice by the limiting dilution technique. In this way, clones for which high antibody titer values are stably obtainable against the ganglioside GD3 are selected as anti-ganglioside GD3 monoclonal antibody-producing hybridoma cell lines.

(4) Preparation of monoclonal antibodies

An anti-ganglioside GD3 monoclonal antibody-producing hybridoma cell line obtained as described above under (3) is cultured in RPMI-1640 medium supplemented with 10% FCS or in serum-free RPMI-1640 medium until cells become confluent, when the culture supernatant is recovered. This supernatant is adjusted to pH 8.9 with sodium hydroxide, and treated with protein A-Sepharose 4B (Pharmacia) for antibody adsorption either chromatographically or batchwise. In the former case, the pH-adjusted culture supernatant is applied to a column packed with protein A-Sepharose 4B. In the case of batchwise treatment, protein A-Sepharose 4B is added to the culture supernatant, and the resulting gel is packed into a column. In either case, the column is washed with a binding buffer (1.5 M glycine, 3 M sodium chloride, pH 8.9) and elution is carried out using an elution buffer (0.1 M citric acid, pH 4.0). IgG fractions are combined, adjusted to pH 7 to 8 with sodium hydroxide, and dialyzed against 2 M sodium chloride-PBS to give a purified monoclonal antibody.

The subclass of the antibody can be determined using a mouse monoclonal antibody typing kit (Zymed Laboratories).

The amount of protein can be determined by the Folin method, followed by calculation based on the optical density at 280 nm [1.0 ($OD_{280}$) being approximately equal to 1 mg immunoglobulin per ml].

The anti-ganglioside GD3 monoclonal antibodies according to the present invention have high reactive specificity and therefore are useful, for instance, in detecting and analyzing GD3 gangliosides. Furthermore, said monoclonal antibodies, as such, have complement-dependent cytotoxic activity (CDC activity) and antibody-dependent cell-mediated cytotoxic activity (ADCC activity) and are expected to exhibit antitumor effects, for example cytocidal effect, on tumor cells expressing the gangliosides GD3 and 3',8'-LD1 by binding to said cells, without impairing normal cells.

The following example are further illustrative of the present invention.

## EXAMPLE 1

### (1) Antigen preparation

A solution of 5 μg of a GD3 ganglioside (Iatron Laboratories) having the carbohydrate chain NeuAcα2→8NeuAcα2→3Gal at the nonreducing end, 0.5 micromole of dipalmitoylphosphatidylcholine (Sigma), 0.5 micromole of cholesterol (Nakalai Tesque), 0.05 micromole of dipalmitoylphosphatidic acid (Sigma) and 2.5 μg of lipid A (Funakoshi Yakuhin) in 30 ml of a 2:1 chloroform-methanol mixture was warmed at 45°C and the solvent was thereby removed to give a thin, uniform lipid film. Suction was effected for 1 hour using a vacuum pump for complete solvent elimination, and 0.5 ml of PBS was then added, followed by stirring at 45°C, whereby an antigen solution was obtained.

### (2) Preparation of antibody-producing cells

Mice were immunized by administering 0.5 ml of the antigen solution prepared as described above under (1) into the caudal vein once every week for 7 weeks. For further immunization, GD3 ganglioside-positive SK-MEL-28 (ATCC HTB72) cells (1 x $10^7$ cells) were intraperitoneally administered once every week for 3 weeks. On the third day after the last administration, splenocytes were prepared from each mouse and submitted for cell fusion.

### (3) Preparation of mouse myeloma cells

The 8-azaguanine-resistant mouse myeloma cell line P3-U1 was cultured in normal medium and not less than 2 x $10^7$ cells were thereby obtained at the time of cell fusion and submitted for cell fusion as a parent strain.

### (4) Hybridoma production

The splenocytes and myeloma cells obtained as described above under (2) and (3), respectively, were used in a ratio of 10:1 and subjected to fusion following the procedure hereinabove. Cultivation was conducted in HAT medium at 37°C for 14 days in the presence of 5% $CO_2$, and fused cells were selected. After changing the medium to HT medium, cultivation was continued. Then, active wells were selected by assaying the antibody titer against ganglioside GD3, and after changing the medium to normal medium, cloning was repeated twice. Hybridomas specifically reactive with ganglioside GD3 were selected by enzyme immunoassay or immunohistological evaluation (ABC method). Thus, 2 ng of ganglioside GM3 [purified from dog erythrocytes by the method of Nores et al. (J. Immunol., 139, 3171 (1987)] or 2 ng of ganglioside GD3 (Iatron) was dissolved in 2 ml ethanol containing 5 ng of phosphatidylcholine (Sigma) and 2.5 ng of cholesterol (Sigma). The resulting solution was distributed in 20-μl portions into wells of a 96-well microtiter plate (Flow Laboratories). After air-drying, blocking was performed with 1% BSA-PBS. The hybridoma culture supernatant was distributed in 50-μl portions into the plate wells carrying the GD3 ganglioside absorbed thereon and the plate wells carrying the GM3 ganglioside adsorbed thereon, and the reaction was allowed to proceed at 4°C for 18 hours.

Thereafter the known reaction procedure described in Cancer Res., 46, 4438 (1986) was followed, and hybridoma strains producing mouse monoclonal antibodies specifically reactive with the GD3 ganglioside but not with the GM3 ganglioside were selected.

Hereinafter, said mouse monoclonal antibodies are referred to as mouse monoclonal antibodies KM-641, KM-643 and KM-644.

(5) Purification of monoclonal antibodies

(a) Preparation of protein A-Sepharose 4B

Cyanogen bromide-activated Sepharose 4B (Pharmacia) (dry powder, 15 g) was allowed to swell in 100 ml of 1 mM hydrochloric acid for 15 minutes and then washed with 3 liters of 1 mM hydrochloric acid. The gel thus obtained was washed with 75 ml of a binding buffer [0.5 M sodium chloride, 0.1 M sodium hydrogen carbonate, pH 8.3] to give a gel suspension.

A solution of 500 mg of protein A (Pharmacia) in the same binding buffer as mentioned above, which had been prepared in advance, was admixed quickly with the above-mentioned gel suspension, and the mixture was stirred gently at room temperature for 2 hours. After stirring, the gel was reacted with 200 ml of a blocking reagent (0.2 M glycine, pH 8.0) at room temperature for 2 hours for blocking remaining active groups. Furthermore, for removing the excessively adsorbed portion of protein, the gel was washed alternately with five 100-ml portions of 0.5 M sodium chloride-0.1 M acetic acid buffer (pH 4.0) and five 100-ml portions of 0.5 M sodium chloride-binding buffer to give 50 ml of protein A-Sepharose 4B (gel).

(b) Recovery of cell culture supernatants

The hybridomas KM-641, KM-643 and KM-644 were respectively cultured at 37°C in a serum-free medium [each liter of medium containing 10.4 g of RPMI-1640 (Nissui Pharmaceutical), 20 ml of 7.5% sodium hydrogen carbonate, 10 mM N-(2-hydroxyethyl)piperazine-N'-2-ethanesulfonic acid (HEPES), 0.29 g of glutamine, 3 mg of insulin (Sigma), 5 µg of transferrin (Sigma), 15 mM sodium pyruvate, 125 nM of sodium selenite, 1 g of galactose, and 0.1% Pepol B-188 (Toho Chiba Chemical)]. When a confluent state was reached, each culture supernatant was recovered and stored frozen at -20°C.

(c) Purification of antibodies from cell culture supernatants

The frozen-stored culture supernatants (60 liters for KM-641, 18 liters for KM-643, and 33 liters for KM-644) were each thawed, adjusted to pH 8.9 with sodium hydroxide, and supplemented with phenylmethylsulfonyl fluoride (PMSF, Nakalai Tesque) and ethylenediaminetetraacetic acid disodium salt (EDTA, Wako Pure Chemical) to final concentrations of 0.1 mM and 5 mM, respectively. After thorough mixing, each mixture was filtered to remove cell debris. To each of the culture supernatants thus processed, 40 ml of the protein A-Sepharose 4B gel prepared as described above under (a) was added, and the mixture was stirred at 4°C for 12 to 20 hours. The gel was recovered from each culture supernatant, packed in a column, and washed with 600 ml of a binding buffer (1.5 M glycine, 3 M sodium chloride, pH 8.9). Elution was then carried out with 200 ml of an elution buffer (0.1 M citric acid, pH 4.0). The eluate was fractioned into 10-ml portions, and IgG fractions were identified by the measurement of optical density at 280 nm ($OD_{280\ nm}$). The IgG fractions were pooled, adjusted to pH 7 to 8 with sodium hydroxide, and dialyzed against 2 M sodium chloride in PBS to give a purified monoclonal antibody.

(6) Subclass identification

The mouse monoclonal antibodies KM-641, KM-643 and KM-644 were shown to belong to the subclass IgG3 by ELISA using a mouse monoclonal antibody typing kit (Zymed).

(7) Specificity testing of KM-641, KM-643 and KM-644

The gangliosides used as standards in testing of KM-641, KM-643 and KM-644 for reactive specificity, namely N-acetylgalactosamine(GalNAc)GM1b, GD1a, GD1b and GT1b, were type V gangliosides purchased from Sigma Chemical, and the gangliosides GD3 and N-acetyl-GM2 were purchased from Iatron Laboratories. The gangliosides GM3, GM1, N-glycolyl-GM2 and GD2 were prepared from dog erythrocytes, bovine brain, mouse liver, and cultured cell line IMR32 (ATCC CCL127), respectively, essentially as described in the literature [Journal of Biological Chemistry, 263, 10915, 2079 (1988)].

The antibodies KM-641, KM-643 and KM-644 were tested for specificity by TLC (thin layer chromatography) immunostaining and enzyme immunoassay as mentioned below.

(a) TLC immunostaining

TLC immunostaining was carried out by the TLC resorcinol (Merck) staining method as essentially described in the literature [J. Biol. Chem., 257, 14365 (1982)]. Thus, the gangliosides GM3, GM2, GM1, GD3, GDla, GalNAcGM1b, 3',8'-LD1 and GT1b were spotted onto HTPLC plates (Whatman) and developed using the developing solvent system chloroform-methanol-0.25% aqueous calcium chloride (50:40:10). The plates were then dipped in a 0.5% solution of poly(isobutyl methacrylate) (Aldrich) in ethanol and, after drying, treated with 1% BSA-PBS for 1 hour to thereby effect blocking. Then, KM-641, KM-643 and KM-644 were used each as a first antibody in the form of a 10 µg/ml solution in 1% BSA-PBS, and incubation was conducted at room temperature for 18 hours. After washing the plates with PBS, biotinylated anti-mouse immunoglobulin antibody (Dako) was added as a second antibody, and incubation was conducted for 1 hour. Further, after washing with PBS, the plates were treated with avidinperoxidase (Dako) for 1 hour, followed by color development with Bio-Rad's HRP color development reagent.

KM-641 and KM-644 reacted with GD3 and 3',8'-LD1, and KM-643 reacted with GD3, 3',8'-LD1, GD1b and GT1b.

(b) Enzyme immunoassay (ELISA)

The gangliosides N-acetyl-GM3, N-glycolyl-GM3, N-acetyl-GM2, N-glycolyl-GM2, GM1, GD3, GD1a, GD2, GD1b, GT1b and GQ1b were used as antigens. They were allowed to be adsorbed on respective wells of 96-well plates for EIA (Flow Laboratories), and their reactivity with KM-641, KM-643 and KM-644 was tested by the ELISA technique mentioned above.

Phosphatidylcholine and cholesterol were used as controls. Ethanol solutions containing them were respectively distributed in 20-µl portions into wells of EIA plates. Then, the same procedure as mentioned above was followed, and optical density measurements were performed.

The results thus obtained with KM-641 are shown in Table 1. KM-641 strongly reacted only with the ganglioside GD3.

Table 1

| Ganglioside | $OD_{415}$ |
|---|---|
| N-Acetyl-GM3 | 0.000 |
| N-Glycolyl-GM3 | 0.001 |
| N-Acetyl-GM2 | 0.000 |
| N-Glycolyl-GM2 | 0.000 |
| GM1 | 0.000 |
| GD3 | 1.112 |
| GD1a | 0.250 |
| GD2 | 0.012 |
| GD1b | 0.039 |
| GT1b | 0.045 |
| GQ1b | 0.003 |
| Control | 0.000 |

KM-643 and KM-644 were tested for reactivity with various gangliosides in the same manner as mentioned above. Control tests were performed in the same manner as mentioned above, followed by measurement of optical density.

The results are shown in Table 2 and Table 3. KM-643 strongly reacted with the gangliosides GD3 and GDlb. KM-644 strongly reacted with the ganglioside GD3.

Table 2

| Ganglioside | $OD_{415}$ |
|---|---|
| N-Acetyl-GM3 | 0.000 |
| N-Glycolyl-GM3 | 0.001 |
| N-Acetyl-GM2 | 0.000 |
| N-Glycolyl-GM2 | 0.000 |
| GM1 | 0.002 |
| GD3 | 1.420 |

Table 2   (continued)

| Ganglioside | $OD_{415}$ |
|---|---|
| GD1a | 0.013 |
| GD2 | 0.085 |
| GD1b | 1.095 |
| GT1b | 0.145 |
| GQ1b | 0.096 |
| Control | 0.000 |

Table 3

| Ganglioside | $OD_{415}$ |
|---|---|
| N-Acetyl-GM3 | 0.000 |
| N-Glycolyl-GM3 | 0.004 |
| N-Acetyl-GM2 | 0.000 |
| N-Glycolyl-GM2 | 0.000 |
| GM1 | 0.000 |
| GD3 | 0.781 |
| GD1a | 0.006 |
| GD2 | 0.001 |
| GD1b | 0.082 |
| GT1b | 0.003 |
| GQ1b | 0.000 |
| Control | 0.000 |

EXAMPLE 2

Reactivity study of KM-641, KM-643 and KM-644 by fluorescent antibody technique

The cultured human malignant melanoma cell lines SK-MEL-28 (ATCC HTB 72), WM266-4 (ATCC CRL1676) and KHm-1/4 [J. Natl. Cancer Inst., 59, 775 (1977)] and the cultured human neuroblastoma cell line IMR32 (ATCC CCLK127) were used. For each cell line, 1 x 10$^6$ cells were placed in a microtube (Treff) and washed by centrifugation (1,200 rpm, 5 minutes) with PBS, 50 $\mu$l of KM-641, KM-643 or KM-644 (10 $\mu$l/ml) was added and, after stirring, incubation was performed at 4°C for 30 minutes. Thereafter, the cells were washed three times by centrifugation (1,200 rpm, 5 minutes) with PBS, then 20 $\mu$l of fluorescein isothiocyanate-labeled anti-mouse IgG antibody and IgM antibody (Cappel Laboratories, 20-fold diluted) was added and, after stirring, the mixture was incubated at 4°C for 30 minutes. Thereafter, the cells were washed three times by centrifugation (1,200 rpm, 5 minutes) with PBS, further suspended in PBS, and submitted for analysis using a Nippon Bunko model FCS-1 flow cell sorter.

In addition, the anti-ganglioside GD3 antibody R24 (MEL-1, Signet) belonging to the subclass IgG3 was used in lieu of KM-641, KM-643 or KM-644 and analysis was made by the same procedure as mentioned above.

Control tests without the addition of KM-641, KM-643 or KM-644 were performed by the same analytical procedure as mentioned above.

The results thus obtained are shown in Fig. 1 and Fig. 2. The fluorescence intensity peaks for KM-641, KM-643 and KM-644 showed shifting to the right (increased fluorescence intensity) as compared with the controls, indicating that these antibodies had reacted directly with glycolipid carbohydrate chains expressed on the surface of various cells although the reactivity differed to some extent among the antibodies. KM-641, KM-643 and KM-644 were stronger in reactivity than the anti-ganglioside GD3 antibody R24.

## EXAMPLE 3

Complement-dependent cytotoxicity (CDC)

(a) Preparation of target cells

Suspensions of the target cells, namely ganglioside GD3-positive G361 cells (JCRB) and SK-MEL-28 cells, in RPMI-1640 medium supplemented with 10% FCS were respectively adjusted to a cell concentration of $1 \times 10^7$ cells/ml, $Na_2{}^{51}CrO_4$ was added to a concentration of 100 µCi/$1 \times 10^7$ cells, incubation was performed at 37°C for 1 hour and, thereafter, the cells were washed three times with the medium. The cells were allowed to stand in the medium at 4°C for 30 minutes for spontaneous dissociation and then centrifuged (1,200 rpm, 5 minutes), and the medium was added to adjust the cell concentration to $4 \times 10^6$ cells/ml.

(b) Complement absorption

To an aqueous solution of lyophilized rabbit complement (Cedarlane Laboratories) were added $2 \times 10^7$ target cells prepared as described above under (a). Incubation was carried out at 4°C for 30 minutes. The suspension was centrifuged (1,200 rpm, 5 minutes), $2 \times 10^7$ target cells were again added, and incubation was conducted in the same manner as mentioned above. Thereafter, the suspension was passed through a filter to remove the cells. The filtrate was used as a complement solution.

(c) CDC activity measurement

To U-bottomed 96-well plates was added KM-641, KM-643 or KM-644 to final concentrations within the range of 0.05 µg/ml to 50 µg/ml. To each well were added $2 \times 10^5$ target cells. Incubation was performed at room temperature for 1 hour. The supernatants were removed by centrifugation (1,200 rpm, 5 minutes), the complement solution prepared as described above under (b) was added in 50-µl portions, and incubation was performed at 37°C for 1 hour. After centrifugation (1,200 rpm, 5 minutes), the amount of $^{51}Cr$ in each supernatant was determined using a gamma-counter. The amount of $^{51}Cr$ resulting from spontaneous dissociation was determined by adding to target cells the medium alone in lieu of the antibody and complement solution and determining the amount of $^{51}Cr$ in the supernatant in the same manner as described above. The total amount of free $^{51}Cr$ was determined by adding 5 N sodium hydroxide in lieu of the complement solution, proceeding as described above, and determining the amount of $^{51}Cr$ in the supernatant.

The CDC activity was calculated as follows:

$$\text{CDC activity (\%)} =$$

$$\frac{\text{Amount of } {}^{51}Cr \text{ in sample supernatant - Amount of } {}^{51}Cr \text{ resulting from spontaneous dissociation}}{\text{Total amount of free } {}^{51}Cr \text{ - Amount of } {}^{51}Cr \text{ resulting from spontaneous dissociation}} \times 100$$

As a control, the medium was used in lieu of the complement solution, the procedure mentioned above was followed, and the amount of $^{51}Cr$ for the control determined for CDC activity calculation.

The results thus obtained are shown in Table 4. KM-641, KM-643 and KM-644 showed strong cytotoxicity against the GD3-positive G361 and SK-MEL-28 cells.

Table 4

| Target cell | Antibody | Antibody concentration (µg/ml) | CDC activity (%) | |
|---|---|---|---|---|
| | | | In the presence of complement | In the absence of complement |
| SK-MEL-28 | KM-641 | 50 | 16.3 | 0.9 |
| | | 5 | 32.4 | 0.0 |
| | | 0.5 | 18.6 | 0.0 |
| | | 0.05 | 2.7 | 0.0 |
| | KM-643 | 50 | 22.6 | 0.2 |
| | | 5 | 29.1 | 0.0 |

Table 4   (continued)

| Target cell | Antibody | Antibody concentration (μg/ml) | CDC activity (%) | |
|---|---|---|---|---|
| | | | In the presence of complement | In the absence of complement |
| G361 | KM-644 | 0.5 | 24.1 | 0.0 |
| | | 0.05 | 6.0 | 0.0 |
| | | 50 | 25.6 | 16.9 |
| | | 5 | 33.5 | 1.7 |
| | KM-641 | 0.5 | 22.9 | 0.0 |
| | | 0.05 | 4.6 | 0.0 |
| | | 50 | 23.5 | 5.8 |
| | | 5 | 47.0 | 1.2 |
| | KM-643 | 0.5 | 32.4 | 2.0 |
| | | 0.05 | 15.2 | 2.7 |
| | | 50 | 64.0 | 0.3 |
| | | 5 | 47.7 | 0.0 |
| | KM-644 | 0.5 | 27.8 | 0.0 |
| | | 0.05 | 10.7 | 0.6 |
| | | 50 | 23.3 | 21.7 |
| | | 5 | 40.7 | 4.2 |
| | | 0.5 | 28.0 | 0.0 |
| | | 0.05 | 12.3 | 0.0 |

EXAMPLE 4

Antibody-dependent cell-mediated cytotoxicity (ADCC)

(a) Preparation of target cells

Suspensions of the target SK-MEL-28 and G361 cells in RPMI-1640 medium supplemented with 10% FCS were each adjusted to a cell concentration of $1 \times 10^6$ cells/0.5 ml, $Na_2{}^{51}CrO_4$ was added to a concentration of 50 μCi/1 x $10^6$ cells, incubation was performed at 37°C for 1 hour, and the cells were washed three times with the medium. The cells were then allowed to stand in the medium at 4°C for 30 minutes for spontaneous dissociation, then separated by centrifugation (1,200 rpm, 5 minutes) and adjusted to $2 \times 10^5$ cells/ml by addition of the medium.

(b) Preparation of effector cells

Human venous blood (50 ml) was collected, 0.5 ml of heparin sodium (Takeda Chemical Industries, 1,000 units/ml) was added, and the mixture was stirred gently and then centrifuged (1,500 to 1,800 g, 15 minutes) using a Leuko-PREP (Becton-Dickinson Japan). The cell layer was separated, and the cells were washed three times by centrifugation (1,500 to 1,800 g, 15 minutes) with PRMI-1640 medium and suspended in RPMI-1640 medium supplemented with 10% FCS ($5 \times 10^6$ cells/ml) for use as effector cells (lymphocytes).

(c) ADCC activity measurement

To U-bottomed 96-well plates was added KM-641, KM-643 or KM-644 in 50-μl portions to final concentrations within the range of 0.05 μg/ml to 50 μg/ml. To each well were added $1 \times 10^4$ target cells (50 μg) and $5 \times 10^5$ effector cells (100 μl). Incubation was performed at 37°C for 4 hours, followed by centrifugation (1,200 rpm, 5 minutes). The amount of $^{51}Cr$ in each supernatant was determined using a gamma-counter. The amount of $^{51}Cr$ resulting from spontaneous dissociation was determined by adding to target cells the medium alone in lieu of the antibody and effector cells and measuring the amount of $^{51}Cr$ in the supernatant in the same manner as described above. The total amount of free $^{51}Cr$ was determined by adding 5 N sodium hydroxide in lieu of the antibody and effector cells, proceeding as described above, and determining the amount of $^{51}Cr$ in the supernatant.

The ADCC activity was calculated as follows:

$$\text{ADCC activity (\%)} =$$

$$\frac{\text{Amount of } ^{51}\text{Cr in sample supernatant - Amount of } ^{51}\text{Cr resulting from spontaneous dissociation}}{\text{Total amount of free } ^{51}\text{Cr - Amount of } ^{51}\text{Cr resulting from spontaneous dissociation}} \times 100$$

As a control, the medium was added in lieu of the effector cells, the procedure mentioned above was followed, and the amount of $^{51}$Cr in the control supernatant was determined for ADCC activity calculation.

The results thus obtained are shown in Table 5. KM-641, KM-643 and KM-644 showed cell-mediated cytotoxicity against the ganglioside GD3-positive SK-MEL-28 and G361 cells, depending upon the concentration of antibody.

Table 5

| Target cell | Antibody | Antibody concentration ($\mu$g/ml) | ADCC activity (%) | |
|---|---|---|---|---|
| | | | In the presence of lymphocytes | In the absence of lymphocytes |
| SK-MEL-28 | KM-641 | 50 | 43.2 | 0.0 |
| | | 5 | 41.8 | 0.0 |
| | | 0.5 | 16.3 | 0.0 |
| | | 0.05 | 8.9 | 0.1 |
| | KM-643 | 50 | 38.6 | 3.7 |
| | | 5 | 43.2 | 1.1 |
| | | 0.5 | 19.7 | 0.8 |
| | | 0.05 | 9.9 | 0.3 |
| | KM-644 | 50 | 19.5 | 0.0 |
| | | 5 | 49.6 | 0.9 |
| | | 0.5 | 31.4 | 1.5 |
| | | 0.05 | 13.8 | 2.6 |
| G361 | KM-641 | 50 | 28.0 | 0.0 |
| | | 5 | 46.0 | 0.0 |
| | | 0.5 | 22.9 | 0.0 |
| | | 0.05 | 16.0 | 0.0 |
| | KM-643 | 50 | 25.1 | 4.0 |
| | | 5 | 40.2 | 0.0 |
| | | 0.5 | 22.3 | 0.0 |
| | | 0.05 | 16.4 | 0.0 |
| | KM-644 | 50 | 7.6 | 0.0 |
| | | 5 | 27.1 | 0.0 |
| | | 0.5 | 30.2 | 0.0 |
| | | 0.05 | 20.3 | 0.0 |

EXAMPLE 5

Therapeutic effect on transplanted tumors

Human melanoma C24·22 cells (2 x 10[7] cells) were intracutaneously transplanted to abdominal parts of Balb/c nu/nu mice (7 animals/group). Monoclonal antibody KM-641 (200 $\mu$g/animal) was intravenously administered into the mice five times starting from the day of the transplantation of the tumor cells (hereinafter test group A) or on the seventh day after the day of the transplantation (hereinafter test group B). To the mice of the control group, anti-Sialyl Le[a] monoclonal antibody AMC-462 (ECACC 86050801) was administered in the same manner as described above. The therapeutic effect on transplanted tumor cells was determined in terms of survival days of mice and tumor size (volume) calculated by the following equation.

$$\text{Tumor size (mm}^3) = 0.4 \times a \times b^2$$

a: major axis
b: minor axis

The results are shown in Fig. 3 and Fig. 4.

A dose-dependent effect was determined from the results obtained by administering AMC-462 to mice of the control group at a dose of 200 µg/animal from the day of the transplantation and administering KM-641 to mice of the test group at a dose of 200 µg/animal or 100 µg/animal. The results are shown in Fig. 5.

As shown in Fig. 3 and Fig. 4, remarkable growth of tumors was observed in the control group, while the growth of tumors was significantly suppressed and survival days were prolonged in the test group A. In the test group B, growth of tumors was significantly suppressed as compared with the control group though no prolongation of survival days was observed. The number of mice in which tumor cells were taken 100 days after the transplantation was 7 animals out of 7 animals in the control group, 7 animals out of 7 animals in the test group B and 4 animals out of 7 animals in the test group A.

As shown in Fig. 5, a dose of 100 µg/animal of KM-641 could effectively suppress the growth of tumors as well as a dose of 200 µg/animal.

As detailedly illustrated hereinabove, the present invention provides monoclonal antibodies of the IgG class which react with the gangliosides GD3 and 3',8'-LD1 and are useful in the treatment of neuroectodermal carcinomas.

## Claims

**Claims for the following Contracting States : AT, BE, CH, LI, DE, DK, FR, GB, IT, LU, NL, SE**

1. A monoclonal antibody which belongs to the subclass IgG3 and is reactive with GD3 ganglioside and simultaneously with 3',8'-LD1.

2. The monoclonal antibody as claimed in Claim 1, wherein said GD3 ganglioside is selected from among GD3 gangliosides having the carbohydrate chain
   NeuAcα2→8NeuAcα2→3Gal, NeuAcα2→8NeuGcα2→3Gal, NeuGcα2→8NeuAcα2→3Gal or NeuGcα2→8NeuGcα2→3Gal.

3. The monoclonal antibody as claimed in Claim 1 as produced by the hybridoma KM-641 (FERM BP-3116), KM-643 (FERM BP-3117) or KM-644 (FERM BP-3118).

4. A hybridoma which is capable of producing a monoclonal antibody belonging to the subclass IgG3 and reactive with GD3 ganglioside and simultaneously with 3',8'-LD1.

5. The hybridoma as claimed in Claim 4, said hybridoma being selected from the group consisting of the hybridomas KM-641 (FERM BP-3116), KM-643 (FERM BP-3117) and KM-644 (FERM BP-3118).

6. Use of a monoclonal antibody according to claims 1 to 3 for the manufacture of a medicament for diagnosing and/ or treating a neuroectodermal tumor.

7. The use according to claim 6, wherein the tumor treated is human melanoma.

8. Use of a monoclonal antibody according to claims 1 to 3 for the manufacture of a medicament for reducing the size of a neuroectodermal tumor.

9. The use according to claim 6, wherein the tumor treated is human melanoma.

10. A process for preparing monoclonal antibodies as defined in one of the claims 1 to 3, comprising the steps of

    a) fusing the splenocytes of a mouse immunized with the GD3 ganglioside or with cancer cells in which the GD3 ganglioside has been expressed and a suitable mouse myeloma cell line,

b) selecting a hybridoma which produces a monoclonal antibody reactive with the GD3 ganglioside and with 3',8'-LD1,

c) culturing said hybridoma or administering said hybridoma to mice to thereby grow the same in the ascites fluid of said mice, and

d) recovering the thus-produced monoclonal antibody from the culture or from the ascites fluid.

11. A pharmaceutical composition comprising an effective amount of at least one monoclonal antibody as defined in one of the claims 1 to 3 optionally in combination with a pharmaceutically acceptable carrier or adjuvant.

12. A process for preparing a pharmaceutical composition as defined in claim 11, comprising the step of providing an effective amount of at least one monoclonal antibody as defined in one of the claims 1 to 3 in a form suitable for administration optionally in combination with a pharmaceutically acceptable carrier or adjuvant.


**Claims for the following Contracting State : ES**

1. A process for preparing monoclonal antibodies belonging to the subclass IgG3 and being reactive with GD3 ganglioside and simultaneously with 3',8'-LD1, which process comprises the steps of

a) fusing the splenocytes of a mouse immunized with the GD3 ganglioside or with cancer cells in which the GD3 ganglioside has been expressed and a suitable mouse myeloma cell line,
b) selecting a hybridoma which produces a monoclonal antibody reactive with the GD3 ganglioside and with 3',8'-LD1,
c) culturing said hybridoma or administering said hybridoma to mice to thereby grow the same in the ascites fluid of said mice, and
d) recovering the thus-produced monoclonal antibody from the culture or from the ascites fluid.

2. The process as claimed in Claim 1, wherein said GD3 ganglioside recognized by the produced antibody is selected from among GD3 gangliosides having the carbohydrate chain NeuAc$\alpha$2$\to$8NeuAc$\alpha$2$\to$3G$\alpha$l, NeuAc$\alpha$2$\to$8NeuGc$\alpha$2$\to$3Gal, NeuGc$\alpha$2$\to$8NeuAc$\alpha$2$\to$3Gal or NeuGc$\alpha$2$\to$8NeuGc$\alpha$2$\to$3Gal.

3. The process as claimed in Claim 1 wherein the antibody is produced by the hybridoma KM-641 (FERM BP-3116), KM-643 (FERN BP-3117) or KM-644 (FERM BP-3118).

4. A process for preparing a hybridoma which is capable of producing a monoclonal antibody belonging to the subclass IgG3 and reactive with GD3 ganglioside and simultaneously with 3',8'-LD1, which process comprises

a) fusing the splenocytes of a mouse immunized with the GD3 ganglioside or with cancer cells in which the GD3 ganglioside has been expressed and a suitable mouse myeloma cell line, and
b) selecting a hybridoma which produces a monoclonal antibody reactive with the GD3 ganglioside and with 3',8'-LD1.

5. The process as claimed in Claim 4, wherein said hybridoma is selected from the group consisting of the hybridomas KM-641 (FERM BP-3116), KM-643 (FERM BP-3117) and KM-644 (FERM BP-3118).

6. Use of a monoclonal antibody prepared according to claims 1 to 3 for the manufacture of a medicament for diagnosing and/or treating a neuroectodermal tumor.

7. The use according to claim 6, wherein the tumor treated is human melanoma.

8. Use of a monoclonal antibody prepared according to claims 1 to 3 for the manufacture of a medicament for reducing the size of a neuroectodermal tumor.

9. The use according to claim 6, wherein the tumor treated is human melanoma.

10. A process for preparing a pharmaceutical composition comprising the step of providing an effective amount of at least one monoclonal antibody as defined in one of the claims 1 to 3 in a form suitable for administration optionally in combination with a pharmaceutically acceptable carrier or adjuvant.

**Claims for the following Contracting State : GR**

1. A monoclonal antibody which belongs to the subclass IgG3 and is reactive with GD3 ganglioside and simultaneously with 3',8'-LD1.

2. The monoclonal antibody as claimed in Claim 1, wherein said GD3 ganglioside is selected from among GD3 gangliosides having the carbohydrate chain NeuAc$\alpha$2→8NeuAc$\alpha$2→3Gal, NeuAc$\alpha$2→8NeuGc$\alpha$2→3Gal, NeuGc$\alpha$2→8NeuAc$\alpha$2→3Gal or NeuGc$\alpha$2→8NeuGc$\alpha$2→3Gal.

3. The monoclonal antibody as claimed in Claim 1 as produced by the hybridoma KM-641 (FERM BP-3116), KM-643 (FERM BP-3117) or KM-644 (FERM BP-3118).

4. A hybridoma which is capable of producing a monoclonal antibody belonging to the subclass IgG3 and reactive with GD3 ganglioside and simultaneously with 3',8'-LD1.

5. The hybridoma as claimed in Claim 4, said hybridoma being selected from the group consisting of the hybridomas KM-641 (FERM BP-3116), KM-643 (FERM BP-3117) and KM-644 (FERM BP-3118).

6. Use of a monoclonal antibody according to claims 1 to 3 for the manufacture of a medicament for diagnosing and/or treating a neuroectodermal tumor.

7. The use according to claim 6, wherein the tumor treated is human melanoma.

8. Use of a monoclonal antibody according to claims 1 to 3 for the manufacture of a medicament for reducing the size of a neuroectodermal tumor.

9. The use according to claim 6, wherein the tumor treated is human melanoma.

10. A process for preparing monoclonal antibodies as defined in one of the claims 1 to 3, comprising the steps of

   a) fusing the splenocytes of a mouse immunized with the GD3 ganglioside or with cancer cells in which the GD3 ganglioside has been expressed and a suitable mouse myeloma cell line,

   b) selecting a hybridoma which produces a monoclonal antibody reactive with the GD3 ganglioside and with 3',8'-LD1,

   c) culturing said hybridoma or administering said hybridoma to mice to thereby grow the same in the ascites fluid of said mice, and

   d) recovering the thus-produced monoclonal antibody from the culture or from the ascites fluid.

11. A process for preparing a pharmaceutical composition comprising the step of providing an effective amount of at least one monoclonal antibody as defined in one of the claims 1 to 3 in a form suitable for administration optionally in combination with a pharmaceutically acceptable carrier or adjuvant.

**Patentansprüche**

**Patentansprüche für folgende Vertragsstaaten : AT, BE, CH, LI, DE, DK, FR, GB, IT, LU, NL, SE**

1. Monoklonaler Antikörper, der der Subklasse IgG3 angehört und gleichzeitig gegenüber GD3-Gangliosid und 3',8'-LD1 reaktiv ist.

2. Monoklonaler Antikörper nach Anspruch 1, wobei das GD3-Gangliosid ausgewählt ist unter GD3-Gangliosiden, die die Kohlenhydratkette NeuAc$\alpha$2$\rightarrow$8NeuAc$\alpha$2$\rightarrow$3Gal, NeuAc$\alpha$2$\rightarrow$8NeuGc$\alpha$2$\rightarrow$3Gal, NeuGc$\alpha$2$\rightarrow$8NeuAc$\alpha$2$\rightarrow$3Gal oder NeuGc$\alpha$2$\rightarrow$8NeuGc$\alpha$2$\rightarrow$3Gal aufweisen.

3. Monoklonaler Antikörper nach Anspruch 1, produziert von Hybridom KM-641 (FERM BP-3116), KM-643 (FERM BP-3117) oder KM-644 (FERM BP-3118).

4. Hybridom, das befähigt ist, einen der Subklasse IgG3 angehörenden und gleichzeitig gegenüber GD3-Gangliosid und 3',8'-LD1 reaktiven Antikörper zu produzieren.

5. Hybridom nach Anspruch 4, wobei das Hybridom ausgewählt ist unter den Hybridomen KM-641 (FERM BP-3116), KM-643 (FERM BP-3117) und KM-644 (FERM BP-3118).

6. Verwendung eines monoklonalen Antikörpers nach einem der Ansprüche 1 bis 3 zur Herstellung eines Medikamentes zur Diagnose und/oder Behandlung eines neuroectodermalen Tumors.

7. Verwendung nach Anspruch 6, wobei der behandelte Tumor ein menschliches Melanom ist.

8. Verwendung eines monoklonalen Antikörpers nach einem der Ansprüche 1 bis 3 zur Herstellung eines Medikamentes zur Verringerung der Größe eines neuroectodermalen Tumors.

9. Verwendung nach Anspruch 6, wobei der behandelte Tumor ein menschliches Melanom ist.

10. Verfahren zur Herstellung monoklonaler Antikörper gemäß einem der Ansprüche 1 bis 3, das folgende Schritte umfasst:

   a) Fusionieren der Splenozyten einer mit dem GD3-Gangliosid oder mit Krebszellen, in denen das GD3-Gangliosid exprimiert worden ist, immunisierten Maus und einer geeigneten Maus-Myelom-Zellinie,
   b) Selektieren eines Hybridoms, das einen monoklonalen Antikörper produziert, der gegenüber dem GD3-Gangliosid und 3',8'-LD1 reaktiv ist,
   c) Kultivieren des Hybridoms oder Verabreichung des Hybridoms an Mäuse, um dieses in der Ascitesflüssigkeit der Mäuse zu züchten, und
   d) Isolierung des so produzierten monoklonalen Antikörpers aus der Kultur oder aus der Ascitesflüssigkeit.

11. Pharmazeutische Zusammensetzung, die eine wirksame Menge wenigstens eines monoklonalen Antikörpers gemäß einem der Ansprüche 1 bis 3, gegebenenfalls zusammen mit einem pharmazeutisch akzeptablen Träger oder Adjuvans, enthält.

12. Verfahren zur Herstellung einer pharmazeutischen Zusammensetzung gemäß Anspruch 11, wobei man eine wirksame Menge wenigstens eines monoklonalen Antikörpers gemäß einem der Ansprüche 1 bis 3 in einer zur Verabreichung geeigneten Form, gegebenenfalls zusammen mit einem pharmazeutisch akzeptablen Träger oder Adjuvans, bereitstellt.

**Patentansprüche für folgenden Vertragsstaat : ES**

1. Verfahren zur Herstellung monoklonaler Antikörper, die der Subklasse IgG3 angehören und gleichzeitig gegenüber GD3-Gangliosid und 3',8'-LD1 reaktiv sind, wobei das Verfahren folgende Schritte umfasst:

   a) Fusionieren der Splenozyten einer mit dem GD3-Gangliosid oder mit Krebszellen, in denen das GD3-Gangliosid exprimiert worden ist, immunisierten Maus und einer geeigneten Maus-Myelom-Zellinie,
   b) Selektieren eines Hybridoms, das einen monoklonalen Antikörper produziert, der gegenüber dem GD3-Gangliosid und 3',8'-LD1 reaktiv ist,
   c) Kultivieren des Hybridoms oder Verabreichung des Hybridoms an Mäuse, um dieses in der Ascitesflüssigkeit der Mäuse zu züchten, und

d) Isolierung des so produzierten monoklonalen Antikörpers aus der Kultur oder aus der Ascitesflüssigkeit.

2. Verfahren nach Anspruch 1, wobei das durch den produzierten Antikörper erkannte GD3-Gangliosid unter GD3-Gangliosiden ausgewählt ist, die die Kohlenhydratkette NeuAc$\alpha$2$\rightarrow$8NeuAc$\alpha$2$\rightarrow$3Gal, NeuAc$\alpha$2$\rightarrow$8NeuGc$\alpha$2$\rightarrow$3Gal, NeuGc$\alpha$2$\rightarrow$8NeuAc$\alpha$2$\rightarrow$3Gal oder NeuGc$\alpha$2$\rightarrow$8NeuGc$\alpha$2$\rightarrow$3Gal aufweisen.

3. Verfahren nach Anspruch 1, wobei der Antikörper von Hybridom KM-641 (FERM BP-3116), KM-643 (FERM BP-3117) oder KM-644 (FERM BP-3118) produziert wird.

4. Verfahren zur Herstellung eines Hybridoms, das befähigt ist, einen der Subklasse IgG3 angehörenden und gleichzeitig gegenüber GD3-Gangliosid und 3',8'-LD1 reaktiven monoklonalen Antikörper zu produzieren, wobei man:

    a) die Splenozyten einer mit dem GD3-Gangliosid oder mit Krebszellen, in denen das GD3-Gangliosid exprimiert worden ist, immunisierten Maus und eine geeignete Maus-Myelom-Zellinie fusioniert, und
    b) ein Hybridom selektiert, das einen monoklonalen Antikörper produziert, der gegenüber dem GD3-Gangliosid und 3',8'-LD1 reaktiv ist.

5. Verfahren nach Anspruch 4, wobei das Hybridom ausgewählt ist unter den Hybridomen KM-641 (FERM BP-3116), KM-643 (FERM BP-3117) und KM-644 (FERM BP-3118).

6. Verwendung eines monoklonalen Antikörpers, der nach einem der Ansprüche 1 bis 3 hergestellt worden ist, zur Herstellung eines Medikamentes zur Diagnose und/oder Behandlung eines neuroectodermalen Tumors.

7. Verwendung nach Anspruch 6, wobei der behandelte Tumor ein menschliches Melanom ist.

8. Verwendung eines monoklonalen Antikörpers, der nach einem der Ansprüche 1 bis 3 hergestellt worden ist, zur Herstellung eines Medikamentes zur Verringerung der Größe eines neuroectodermalen Tumors.

9. Verwendung nach Anspruch 6, wobei der behandelte Tumor ein menschliches Melanom ist.

10. Verfahren zur Herstellung einer pharmazeutischen Zusammensetzung, wobei man eine wirksame Menge von wenigstens einem monoklonalen Antikörper gemäß einem der Ansprüche 1 bis 3 in einer zur Verabreichung geeigneten Form, gegebenenfalls zusammen mit einem pharmazeutisch akzeptablen Träger oder Adjuvans, bereitstellt.


**Patentansprüche für folgenden Vertragsstaat : GR**

1. Monoklonaler Antikörper, der der Subklasse IgG3 angehört und gleichzeitig gegenüber GD3-Gangliosid und 3',8'-LD1 reaktiv ist.

2. Monoklonaler Antikörper nach Anspruch 1, wobei das GD3-Gangliosid ausgewählt ist unter GD3-Gangliosiden, die die Kohlenhydratkette NeuAc$\alpha$2$\rightarrow$8NeuAc$\alpha$2$\rightarrow$3Gal, NeuAc$\alpha$2$\rightarrow$8NeuGc$\alpha$2$\rightarrow$3Gal, NeuGc$\alpha$2$\rightarrow$8NeuAc$\alpha$2$\rightarrow$3Gal oder NeuGc$\alpha$2$\rightarrow$8NeuGc$\alpha$2$\rightarrow$3Gal aufweisen.

3. Monoklonaler Antikörper nach Anspruch 1, produziert von Hybridom KM-641 (FERM BP-3116), KM-643 (FERM BP-3117) oder KM-644 (FERM BP-3118).

4. Hybridom, das befähigt ist, einen der Subklasse IgG3 angehörenden und gleichzeitig gegenüber GD3-Gangliosid und 3',8'-LD1 reaktiven Antikörper zu produzieren.

5. Hybridom nach Anspruch 4, wobei das Hybridom ausgewählt ist unter den Hybridomen KM-641 (FERM BP-3116), KM-643 (FERM BP-3117) und KM-644 (FERM BP-3118).

6. Verwendung eines monoklonalen Antikörpers nach einem der Ansprüche 1 bis 3 zur Herstellung eines Medikamentes zur Diagnose und/oder Behandlung eines neuroectodermalen Tumors.

**7.** Verwendung nach Anspruch 6, wobei der behandelte Tumor ein menschliches Melanom ist.

**8.** Verwendung eines monoklonalen Antikörpers nach einem der Ansprüche 1 bis 3 zur Herstellung eines Medikamentes zur Verringerung der Größe eines neuroectodermalen Tumors.

**9.** Verwendung nach Anspruch 6, wobei der behandelte Tumor ein menschliches Melanom ist.

**10.** Verfahren zur Herstellung monoklonaler Antikörper gemäß einem der Ansprüche 1 bis 3, das folgende Schritte umfasst:

a) Fusionieren der Splenozyten einer mit dem GD3-Gangliosid oder mit Krebszellen, in denen das GD3-Gangliosid exprimiert worden ist, immunisierten Maus und einer geeigneten Maus-Myelom-Zellinie,
b) Selektieren eines Hybridoms, das einen monoklonalen Antikörper produziert, der gegenüber dem GD3-Gangliosid und 3',8'-LD1 reaktiv ist,
c) Kultivieren des Hybridoms oder Verabreichung des Hybridoms an Mäuse, um dieses in der Ascitesflüssigkeit der Mäuse zu züchten, und
d) Isolierung des so produzierten monoklonalen Antikörpers aus der Kultur oder aus der Ascitesflüssigkeit.

**11.** Verfahren zur Herstellung einer pharmazeutischen Zusammensetzung, wobei man eine wirksame Menge wenigstens eines monoklonalen Antikörpers gemäß einem der Ansprüche 1 bis 3 in einer zur Verabreichung geeigneten Form,gegebenenfalls zusammen mit einem pharmazeutisch akzeptablen Träger oder Adjuvans, bereitstellt.

## Revendications

### Revendications pour les Etats contractants suivants : AT, BE, CH, LI, DE, DK, FR, GB, IT, LU, NL, SE

**1.** Anticorps monoclonal appartenant à la sous-classe IgG3 et réagissant avec le ganglioside GD3 et simultanément avec 3',8'-LD1.

**2.** Anticorps monoclonal selon la revendication 1, où ledit ganglioside GD3 est sélectionné parmi des gangliosides GD3 ayant la chaîne hydrate de carbone
NeuAc$\alpha$2$\rightarrow$8 NeuAc$\alpha$2$\rightarrow$3Gal, NeuAc$\alpha$2$\rightarrow$8 NeuGc$\alpha$2$\rightarrow$3Gal, NeuGc$\alpha$$\rightarrow$8 NeuAc$\alpha$2$\rightarrow$3Gal ou NeuGc$\alpha$2$\rightarrow$8 NeuGc$\alpha$2$\rightarrow$3Gal.

**3.** Anticorps monoclonal selon la revendication 1, tel que produit par l'hybridome KM-641 (FERM BP-3116), KM-643 (FERM BP-31117) ou KM-644 (FERM-3118).

**4.** Hybridome capable de produire un anticorps monoclonal appartenant à la sous-classe IgG3 et réagissant avec le ganglioside GD3 et simultanément avec 3',8'-LD1.

**5.** Hybridome selon la revendication 4, ledit hybridome étant sélectionné dans le groupe constitué par les hybridomes KM-641 (FERM BP-3116), KM-643 (FERM BP-3117) et KM-644 (FERM BP-3118).

**6.** Utilisation d'un anticorps monoclonal selon les revendications 1 à 3 pour la préparation d'un médicament destiné au diagnostic et/ou au traitement d'une tumeur neuroectodermique.

**7.** Utilisation selon la revendication 6, où la tumeur traitée est un mélanome humain.

**8.** Utilisation d'un anticorps monoclonal selon les revendications 1 à 3 pour la préparation d'un médicament destiné à réduire la grosseur d'une tumeur neuroectodermique.

**9.** Utilisation selon la revendication 6, où la tumeur traitée est un mélanome humain.

**10.** Procédé de préparation d'anticorps monoclonaux selon les revendications 1 à 3, comprenant les étapes consistant à

17

(a) fusionner les splénocytes d'une souris immunisée par le ganglioside GD3 ou par des cellules cancéreuses dans lesquelles le ganglioside GD3 a été exprimé et une lignée cellulaire appropriée de myélome de souris,
(b) sélectionner un hybridome qui produit un anticorps monoclonal réagissant avec le ganglioside GD3 et avec 3',8'-LD1,
(c) cultiver ledit hybridome ou administrer ledit hybridome à des souris pour le cultiver dans le liquide ascitique desdites souris et
(d) récupérer l'anticorps monoclonal ainsi produit dans la culture ou le liquide ascitique.

11. Composition pharmaceutique comprenant une quantité efficace d'au moins un anticorps monoclonal selon une des revendications 1 à 3, facultativement en combinaison avec un véhicule ou adjuvant pharmaceutiquement acceptable.

12. Procédé de préparation d'une composition pharmaceutique selon la revendication 11, comprenant l'étape consistant à fournir une quantité efficace d'au moins un anticorps monoclonal selon une des revendications 1 à 3 sous une forme convenant à l'administration, facultativement en combinaison avec un véhicule ou adjuvant pharmaceutiquement acceptable.


**Revendications pour l'Etat contractant suivant : ES**

1. Procédé de préparation d'anticorps monoclonaux appartenant à la sous-classe IgG3 et réagissant avec le ganglioside GD3 et simultanément avec 3',8'-LD1, lequel procédé comprend les étapes consistant à

   (a) fusionner les splénocytes d'une souris immunisée par le ganglioside GD3 ou par des cellules cancéreuses dans lesquelles le ganglioside GD3 a été exprimé et une lignée cellulaire appropriée de myélome de souris,
   (b) sélectionner un hybridome qui produit un anticorps monoclonal réagissant avec le ganglioside GD3 et avec 3',8'-LD1,
   (c) cultiver ledit hybridome ou administrer ledit hybridome à des souris pour le cultiver dans le liquide ascitique desdites souris et
   (d) récupérer l'anticorps monoclonal ainsi produit dans la culture ou le liquide ascitique.

2. Procédé selon la revendication 1, où ledit ganglioside GD3 reconnu par l'anticorps produit est sélectionné parmi des gangliosides GD3 ayant la chaîne hydrate de carbone
   NeuAc$\alpha$2$\rightarrow$8NeuAc$\alpha$2$\rightarrow$3Gal, NeuAc$\alpha$2$\rightarrow$8NeuGc$\alpha$2$\rightarrow$3Gal, NeuGc$\alpha$2$\rightarrow$8NeuAc$\alpha$2$\rightarrow$3Gal ou NeuGc$\alpha$2$\rightarrow$8NeuGc$\alpha$2$\rightarrow$3Gal.

3. Procédé selon la revendication 1, où l'anticorps est produit par l'hybridome KM-641 (FERM BP-3116), KM-643 (FERM BP-3117) ou KM-644 (FERM BP-3118).

4. Procédé de préparation d'un hybridome qui est capable de produire un anticorps monoclonal appartenant à la sous-classe IgG3 et qui réagit avec le ganglioside GD3 et simultanément avec 3',8'-LD1, lequel procédé comprend les étapes consistant à

   (a) fusionner les splénocytes d'une souris immunisée par le ganglioside GD3 ou par des cellules cancéreuses dans lesquelles le ganglioside GD3 a été exprimé et une lignée cellulaire appropriée de myélome de souris et
   (b) sélectionner un hybridome qui produit un anticorps monoclonal réagissant avec le ganglioside GD3 et avec 3',8'-LD1.

5. Procédé selon la revendication 4, où ledit hybridome est sélectionné dans le groupe constitué par les hybridomes KM-641 (FERM BP-3116), KM-643 (FERM-3117) et KM-644 (FERM BP-3118).

6. Utilisation d'un anticorps monoclonal préparé selon les revendications 1 à 3 pour la préparation d'un médicament destiné au diagnostic et/ou au traitement d'une tumeur neuroectodermique.

7. Utilisation selon la revendication 6, où la tumeur traitée est un mélanome humain.

8. Utilisation d'un anticorps monoclonal préparé selon les revendications 1 à 3 pour la préparation d'un médicament destiné à réduire la grosseur d'une tumeur neuroectodermique.

**9.** Utilisation selon la revendication 6, où la tumeur traitée est un mélanome humain.

**10.** Procédé de préparation d'une composition pharmaceutique comprenant l'étape consistant à fournir une quantité efficace d'au moins un anticorps monoclonal selon une des revendications 1 à 3 sous une forme convenant à l'administration, facultativement en combinaison avec un véhicule ou adjuvant pharmaceutiquement acceptable.

**Revendications pour l'Etat contractant suivant : GR**

**1.** Anticorps monoclonal appartenant à la sous-classe IgG3 et réagissant avec le ganglioside GD3 et simultanément avec 3',8'-LD1.

**2.** Anticorps monoclonal selon la revendication 1, où ledit ganglioside GD3 est sélectionné parmi des gangliosides GD3 ayant la chaîne hydrate de carbone
NeuAc$\alpha$2$\rightarrow$8NeuAc$\alpha$2$\rightarrow$3Gal, NeuAc$\alpha$2$\rightarrow$8NeuGc$\alpha$2$\rightarrow$3Gal, NeuGc$\alpha$2$\rightarrow$8NeuAc$\alpha$2$\rightarrow$3Gal ou NeuGc$\alpha$2$\rightarrow$8NeuGc$\alpha$2$\rightarrow$3Gal.

**3.** Anticorps monoclonal selon la revendication 1, tel que produit par l'hybridome KM-641 (FERM BP-3116), KM-643 (FERM BP-3117) ou KM-644 (FERM-3118).

**4.** Hybridome capable de produire un anticorps monoclonal appartenant à la sous-classe IgG3 et réagissant avec le ganglioside GD3 et simultanément avec 3',8'-LD1.

**5.** Hybridome selon la revendication 4, ledit hybridome étant sélectionné dans le groupe constitué par les hybridomes KM-641 (FERM BP-3116), KM-643 (FERM BP-3117) et KM-644 (FERM BP-3118).

**6.** Utilisation d'un anticorps monoclonal selon les revendications 1 à 3 pour la préparation d'un médicament destiné au diagnostic et/ou au traitement d'une tumeur neuroectodermique.

**7.** Utilisation selon la revendication 6, où la tumeur traitée est un mélanome humain.

**8.** Utilisation d'un anticorps monoclonal selon les revendications 1 à 3 pour la préparation d'un médicament destiné à réduire la grosseur d'une tumeur neuroectodermique.

**9.** Utilisation selon la revendication 6, où la tumeur traitée est un mélanome humain.

**10.** Procédé de préparation d'anticorps monoclonaux selon les revendications 1 à 3, comprenant les étapes consistant à

(a) fusionner les splénocytes d'une souris immunisée par le ganglioside GD3 ou par des cellules cancéreuses dans lesquelles le ganglioside GD3 a été exprimé et une lignée cellulaire appropriée de myélome de souris,
(b) sélectionner un hybridome qui produit un anticorps monoclonal réagissant avec le ganglioside GD3 et avec 3',8'-LD1,
(c) cultiver ledit hybridome ou administrer ledit hybridome à des souris pour le cultiver dans le liquide ascitique desdites souris et
(d) récupérer l'anticorps monoclonal ainsi produit dans la culture ou le liquide ascitique.

**11.** Procédé de préparation d'une composition pharmaceutique comprenant l'étape consistant à fournir une quantité efficace d'au moins un anticorps monoclonal selon une des revendications 1 à 3 sous une forme convenant à l'administration, facultativement en combinaison avec un véhicule ou adjuvant pharmaceutiquement acceptable.

Fig. 1

K H m − 1 ／ 4

( a )　　( b )　　( c )　　( d )

I M R 3 2

Fig. 2

SK-MEL-28

WM266-4

EP 0 493 686 B1

Fig. 3

Fig. 4

Fig. 5